# EUROPEAN PATENT APPLICATION

(11) **EP 2 505 174 A1**
(43) Date of publication of application: **03.10.2012**
(21) Application number: 11160885.7
(22) Date of filing: 01.04.2011
(51) Int. Cl.: A61F 13/56

(54) **ABSORBENT GARMENT**

(71) Applicant: The Procter & Gamble Company, Cincinnati, OH 45202 (US)
(72) Inventor: VEGLIO', Paolo, 65127, PESCARA (IT)
(74) Representative: Briatore, Andrea

(57) **Abstract**

An absorbent garment comprising first and second waist regions and a crotch region lying longitudinally therebetween is disclosed. The garment has a body-facing surface for facing a wearer and a garment-facing surface for facing away from the wearer. The second waist region extends transversely beyond the crotch region to form terminal portions. Each terminal portion of the second waist region is folded on at least two longitudinally-extending fold lines towards one of the body-facing and garment-facing surfaces of the garment, an outermost adjacent pair of the fold lines dividing the terminal portions into proximal, middle and distal sections, the distal section lying between the proximal and middle sections. At least one closure member for fastening to the first waist region is attached to the distal section of each terminal portion.

## Description

### FIELD OF THE INVENTION

This invention relates to an absorbent garment, such as a diaper, and to methods of using such a garment.

### BACKGROUND OF THE INVENTION

Absorbent garments, such as diapers, for incontinent wearers are well known. In a typical design, an absorbent core is provided between a top sheet and a backing sheet. A crotch region of the garment extends between front and back waist regions. The front and back waist regions extend transversely beyond the crotch region to form ears or wings. Adhesive or hook-and-loop type fasteners are provided (normally on the back waist region) to fasten the front and back waist regions together around a wearer's waist.

Although such an absorbent garment of a particular size will fit a range of waist sizes, it is necessary to produce several different sizes to span the whole range of waist sizes that such garments might be required to fit. Furthermore, when a garment of a particular size is fitted to a wearer having a waist size smaller than the maximum intended for the garment, it is inevitable that the front and back waist regions will have to be overlapped to achieve a fit. The presence of extra material layers at the side of a wearer's body in the region of overlap between the front and back waist regions can lead to a decrease in wearer comfort.

Additionally, under certain circumstances it is possible for the integrity of the closure members to be compromised, for example, if they become snared in the production machinery or during the packaging process, whilst stored or in transit. Such a compromise to the integrity of a closure member can result in it not being able to fulfil its function of fastening the front and rear waist regions.

### SUMMARY OF THE INVENTION

According to the present invention, there is provided an absorbent garment comprising first and second waist regions and a crotch region lying longitudinally therebetween, the garment having a body-facing surface for facing a wearer and a garment-facing surface for facing away from the wearer, wherein the second waist region extends transversely beyond the crotch region to form terminal portions, each terminal portion of the second waist region being folded on at least two longitudinally-extending fold lines towards one of the body-facing and garment-facing surfaces of the garment, an outermost adjacent pair of fold lines dividing the terminal portions into proximal, middle and distal sections, the distal section lying between the proximal and middle sections, wherein at least one closure member for fastening to the first waist region is attached to the distal section of each terminal portion.

According to the present invention, there is also provided an absorbent garment comprising first and second waist regions and a crotch region lying longitudinally therebetween, the garment having a body-facing surface for facing a wearer and a garment-facing surface for facing away from the wearer, wherein the first waist region extends transversely beyond the crotch region to form terminal portions and the second waist region has closure members for fastening to the terminal portions of the first waist region, each terminal portion of the first waist region being folded on at least two longitudinally-extending fold lines towards the body-facing surface of the garment and being divided into proximal, middle and distal sections by an outermost adjacent pair of the fold lines such that, when a terminal portion is unfolded on a first one of the pair of fold lines, a closure member can be fastened directly to the garment-facing surface on the middle section of the unfolded terminal portion, and when a terminal portion is unfolded on both of the pair of fold lines, a closure member can be fastened directly to the garment-facing surface on the distal section of the unfolded terminal portion.

Methods of using the absorbent garment of the present invention are also provided.

### BRIEF DESCRIPTION OF THE DRAWINGS

Figure 1 shows a perspective view of an absorbent garment according to the invention;
Figures 2a to 2c show cross-sections through a terminal portion of a front waist region of the absorbent garment; and
Figures 3 to 7 show the absorbent garment of Figure 1 in various states as the terminal portions referred to above are unfolded.

### DETAILED DESCRIPTION OF THE INVENTION

In accordance with a first aspect of the present invention, there is provided an absorbent garment comprising first and second waist regions and a crotch region lying longitudinally therebetween, the garment having a body-facing surface for facing a wearer and a garment-facing surface for facing away from the wearer, wherein the second waist region extends transversely beyond the crotch region to form terminal portions, each terminal portion of the second waist region being folded on at least two longitudinally-extending fold lines towards one of the body-facing and garment-facing surfaces of the garment, an outermost adjacent pair of fold lines dividing the terminal portions into proximal, middle and distal sections, the distal section lying between the proximal and middle sections, wherein at least one closure member for fastening to the first waist region is attached to the distal section of each terminal portion.

By folding the terminal portions of the second waist region in this way, the closure members are enveloped within the folded terminal portions. This provides an additional degree of protection for the closure members during manufacture and subsequently, which in turn helps ensure that the integrity and functionality of the closure members is not compromised. This protection is enhanced, in particular, because the folding configuration helps to prevent the terminal portions from unravelling, which can occur with a concertina configuration of folding.

The adjacent pair of fold lines referred to above are normally made by folding the terminal portion at a first, distal fold line towards a selected one of the body-facing and garment-facing surfaces of the garment (and towards the longitudinal centreline of the garment) followed by folding the already-folded portion at a second, proximal fold line (closer to the longitudinal centreline of the garment than the first, distal fold line) towards the selected one of the body-facing and garment-facing surfaces of the garment.

By "adjacent" fold lines, we mean that there are no other fold lines on the terminal portions between the pair of adjacent fold lines. The result of folding on two adjacent fold lines is that the terminal portions are divided into three sections: a proximal section (that is, closest to the longitudinal centreline of the garment), a middle section, and a distal section (that is, furthest from the longitudinal centreline of the garment). After folding, the distal section will typically lie between and face-to-face with both the middle and proximal sections, preferably with nothing between the distal section and each of the middle and proximal sections.

It is important that the at least two longitudinally-extending fold lines are made by folding towards a selected one of the body-facing and garment-facing surfaces of the garment. A combination of folding towards the body-facing and garment-facing surfaces cannot be used; all folds must be made in the same direction. The usual configuration is that the at least two longitudinally-extending fold lines are made by folding towards the body-facing surface of the garment.

The divided sections may have the same or substantially the same dimension in the transverse direction as each other. Thus, the distal edges (that is, furthest edges from the longitudinal centreline of the garment) of the terminal portions may be aligned or substantially aligned with a fold line.

A distal edge of each terminal portion of the second waist region may lie between the adjacent pair of fold lines.

The closure members can be folded on the distal edge of the terminal portions of the second waist region towards the body-facing surface of the garment. The closure members may be fastener tapes.

The distal section of each terminal portion of the second waist region can be releasably bonded to the middle and/or proximal sections.

According to the present invention, the distal section of each terminal portion of the second waist region can be releasably bonded to the middle and/or proximal sections in two regions spaced apart in a longitudinal direction to leave an unbonded region therebetween. A user is therefore easily able to grasp the terminal portion by inserting their fingers into the unbonded region to release the bonds.

These releasable bonds may be formed by a low-strength adhesive or by an ultrasonic or other welding process, for example. The releasable bonds may have a relatively small surface area. For example, they may be points of adhesive or spot welds. The releasable bonds ensure that the terminal portions remain folded until it is desired to unfold them. This is particularly advantageous during manufacture as it prevents the terminal portions from inadvertently unfolding and becoming ensnared in the manufacturing machinery. In alternative embodiments, the terminal portions may be maintained in their folded state during manufacture by suction, applied (for example) through holes in a conveyor belt on which the garment is carried along a manufacturing line.

In accordance with a second aspect of the invention, there is provided an absorbent garment comprising first and second waist regions and a crotch region lying longitudinally therebetween, the garment having a body-facing surface for facing a wearer and a garment-facing surface for facing away from the wearer, wherein the first waist region extends transversely beyond the crotch region to form terminal portions and the second waist region has closure members for fastening to the terminal portions of the first waist region, each terminal portion of the first waist region being folded on at least two longitudinally-extending fold lines towards the body-facing surface of the garment and being divided into proximal, middle and distal sections by an outermost adjacent pair of the fold lines such that, when a terminal portion is unfolded on a first one of the pair of fold lines, a closure member can be fastened directly to the garment-facing surface on the middle section of the unfolded terminal portion, and when a terminal portion is unfolded on both of the pair of fold lines, a closure member can be fastened directly to the garment-facing surface on the distal section of the unfolded terminal portion.

By providing a garment in which each terminal portion of the first waist region is folded on an outermost pair of adjacent longitudinally-extending fold lines towards the body-facing surface of the garment, it is possible to vary the degree of transverse extension of the terminal portions when the garment is put on a wearer. The terminal portions may be left folded for small wearers, unfolded once on one of the pair of fold lines for medium-sized wearers, or unfolded twice on both of the pair of fold lines for large wearers.

Furthermore, it is possible to combine the features of the first and second aspects of the invention, whereby terminal portions are provided on each of the first and second waist regions. Thus, it is possible to adjust the degree of transverse extension of the terminal portions beyond the crotch region so that there is optimal overlap at the side of the wearer's body between the terminal portions on the first and second waist regions, thereby improving the wearer's comfort. Yet another advantage of the presence of the pair of adjacent fold lines on the terminal portions is that, when the terminal portions are unfolded either on one or both lines, they may be used as a reference mark for placing the closure members. This helps to ensure a symmetrical fit.

The outermost pair of fold lines referred to above with reference to the second aspect of the invention are normally made by folding the terminal portion at a first, distal fold line towards the body-facing surface of the garment (and towards the longitudinal centreline of the garment) followed by folding the already-folded portion at a second, proximal fold line (closer to the longitudinal centreline of the garment than the first, distal fold line) towards the body-facing surface of the garment.

By "adjacent" fold lines, we mean that there are no other fold lines on the terminal portions between the outermost pair of fold lines. The result of the folding on two adjacent fold lines is that the terminal portions are divided into three sections: a proximal section (that is, closest to the longitudinal centreline of the garment), a middle section, and a distal section (that is, furthest from the longitudinal centreline of the garment). After folding, the distal section will typically lie between and face-to-face with both the middle and proximal sections, preferably with nothing between the distal section and each of the middle and proximal sections.

The divided sections may have the same or substantially the same dimension in the transverse direction as each other. Thus, the distal edges (that is, furthest edges from the longitudinal centreline of the garment) of the terminal portions may be aligned or substantially aligned with a fold line.

A distal edge of each terminal portion of the first waist region may lie between the adjacent pair of fold lines.

According to the present invention, the distal section of each terminal portion of the first waist region can be releasably bonded to the middle and/or proximal sections.

According to the present invention, the distal section of each terminal portion of the first waist region can be releasably bonded to the middle and/or proximal sections in two regions spaced apart in a longitudinal direction to leave an unbonded region therebetween. A user is therefore easily able to grasp the terminal portion by inserting their fingers into the unbonded region to release the bonds.

These releasable bonds may be formed by a low-strength adhesive or by an ultrasonic or other welding process, for example. The releasable bonds may have a relatively small surface area. For example, they may be points of adhesive or spot welds. The releasable bonds ensure that the terminal portions remain folded until it is desired to unfold them. This is particularly advantageous during manufacture as it prevents the terminal portions from inadvertently unfolding and becoming ensnared in the manufacturing machinery. In alternative embodiments, the terminal portions may be maintained in their folded state during manufacture by suction, applied (for example) through holes in a conveyor belt on which the garment is carried along a manufacturing line.

It is possible to combine the features of the first and second aspects of the invention, which results in an absorbent garment having folded terminal portions on both the first and second waist regions.

In a normal arrangement in accordance with either of the first or the second aspect or a combination of them, the first waist region is a front waist region and the second waist region is a back waist region.

In accordance with either of the first or the second aspect or a combination of them, the first waist region and/or the second waist region can be manufactured from a stretchable material.

In accordance with either of the first or the second aspect or a combination of them, each of the terminal portions can be provided as a discrete component that is attached to the first or second waist region. This attachment may be by way of adhesive or by an ultrasonic or other welding process, for example.

In accordance with either of the first or second aspect or a combination of them, each of the terminal portions can be folded along a respective longitudinally-extending line towards the body-facing surface to lie over a portion of the first or second waist region that does not extend transversely beyond the crotch region. This renders the garment more compact, which reduces the size of packaging required to store and transport the garment, and also prevents the folded terminal portions from becoming ensnared in manufacturing machinery. The longitudinally-extending lines can be aligned with respective transverse edges of the crotch region of the garment.

In accordance with either of the first or second aspect or a combination of them, the garment can be folded about at least one transversely-extending fold line. This makes it more compact in the longitudinal direction, which helps to reduce the size of packaging required.

In accordance with either of the first or second aspect or a combination of them, the terminal portions of the first and/or second waist region can be folded on only two longitudinally-extending fold lines. In other words, the distal fold line is an outermost fold line and the proximal fold line is an innermost fold line.

However, in accordance with either of the first or second aspect or a combination of them, the terminal portions of the first and/or second waist region may be folded on more than two longitudinally-extending fold lines.

In accordance with either of the first or second aspect or a combination of them, the body-facing surface can be defined by a liquid permeable top sheet, the garment-facing surface can be defined by a backing sheet that is impervious to liquids, and an absorbent core can be disposed between the top sheet and the backing sheet.

In accordance with a third aspect of the invention, there is provided a method of using an absorbent garment according to the first aspect of the invention, the method comprising placing the second waist region of the garment against a wearer's back, drawing the first waist region of the garment between the wearer's legs to leave the crotch region of the garment between the wearer's legs, placing the first waist region of the garment against a wearer's front, unfolding each terminal portion of the second waist region on each of the pair of adjacent longitudinally-extending fold lines, and fastening the closure members to the first waist region.

In accordance with a fourth aspect of the invention, there is provided a method of using an absorbent garment according to the second aspect of the invention, the method comprising placing the second waist region of the garment against a wearer's back, drawing the first waist region of the garment between the wearer's legs to leave the crotch region of the garment between the wearer's legs, placing the first waist region of the garment against a wearer's front, unfolding each terminal portion of the first waist region on a first one of the pair of adjacent longitudinally-extending fold lines, and fastening the closure members to the first waist region.

The method may further comprise unfolding each terminal portion of the first waist region on a second one of the pair of adjacent longitudinally-extending fold lines prior to fastening the closure members to the first waist region.

The invention will now be described with reference to the accompanying drawings.

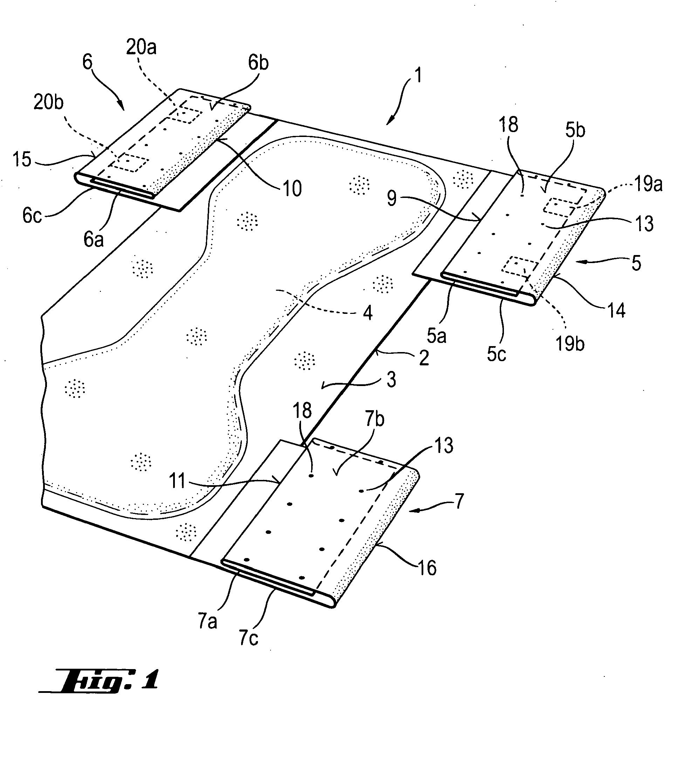

Figure 1 shows an absorbent article, diaper 1, which includes a liquid impervious outer backing sheet 2, a liquid permeable top sheet 3 and an absorbent body or core 4, which may be positioned between the backing sheet 2 and the top sheet 3. The top sheet 3 and backing sheet 2 extend beyond the edges of the absorbent core 4 to thereby form the periphery of a containment assembly.

The backing sheet 2 is impervious to liquids (for example, urine) and may be manufactured from a thin plastic film, although other flexible liquid-impervious materials may also be used. As used herein, the term "flexible" refers to materials that are compliant and will readily conform to the general shape and contours of the human body. The backing sheet 2 prevents liquids absorbed and contained in the absorbent core 4 from wetting articles that contact the diaper 1 such as bed sheets and undergarments. The backing sheet 2 may thus comprise a woven or nonwoven material, polymeric films such as thermoplastic films of polyethylene or polypropylene, or composite materials such as a film-coated nonwoven material. The backing sheet 2 may permit vapours to escape from the absorbent core 4 (that is, it may be breathable) while still preventing liquids from passing through.

The backing sheet 2 is positioned adjacent the garment-facing surface of the absorbent core 4 and can be joined thereto by attachment means (not shown) which are well known in the art. For example, the backing sheet 2 may be secured to the absorbent core 4 by a uniform continuous layer of adhesive, a patterned layer of adhesive, or an array of separate lines, spirals, or spots of adhesive. Alternatively, the attachment means may comprise heat bonds, pressure bonds, ultrasonic bonds, dynamic mechanical bonds, or any other suitable attachment means or combinations of these attachment means as are known in the art.

The top sheet 3 may be compliant, soft feeling, and non-irritating to the wearer's skin. Further, the top sheet 3 is liquid permeable, permitting liquids (for example, urine) to readily penetrate through its thickness. A suitable top sheet 3 may be manufactured from a wide range of materials, such as porous foams, reticulated foams, apertured plastic films, or woven or nonwoven webs of natural fibres (for example, wood or cotton fibres), synthetic fibres (for example, polyester or polypropylene fibres), or a combination of natural and synthetic fibres. The top sheet 3 can be made of a hydrophobic material to isolate the wearer's skin from liquids contained in the absorbent core 4 that is treated on at least one side with a surfactant to allow liquids to readily penetrate through its thickness. When the top sheet comprises a nonwoven web, the web may be spunbonded, carded, wet laid, melt-blown, hydroentangled, or formed under a combination of the above.

The absorbent core 4 may be any absorbent means which is capable of absorbing and retaining liquids such as urine. The absorbent core 4 may be manufactured from a wide variety of liquid absorbent materials commonly used in disposable diapers and other absorbent articles, such as comminuted wood pulp which is often referred to in the absorbent article manufacturing industry as airfelt. Examples of other suitable absorbent materials include creped cellulose wadding, melt-blown polymer fibres or mixtures thereof including coform, chemically modified or cross-linked cellulosic fibres, tissue including tissue wraps and tissue laminates, absorbent foams, absorbent sponges, superabsorbent polymers, absorbent gelling materials, or any equivalent material or combination of materials. The configuration and construction of the absorbent core may also be varied (for example, the absorbent core 4 may have varying caliper zones, hydrophilic gradients, superabsorbent gradients, or lower average density and/or lower average basis weight acquisition zones; or may comprise one or more layers or structures). The total absorbent capacity of the absorbent core 4 should, however, be compatible with the design loading and the intended use of the absorbent article. Further, the size and the absorbent capacity of the absorbent core may be varied to accommodate wearers ranging from infants through adults.

The diaper 1 is arranged in three regions: a front waist region, a back waist region and a crotch region extending longitudinally between the front and back waist regions. Terminal portions (for example, made from the same material as the backing sheet 2) are attached to each of the front and back waist regions, and these terminal portions extend transversely beyond the edge of the crotch region. Thus, the back waist region has two terminal portions 5, 6 and the front waist region has two terminal portions 7, 8.

The terminal portions 5, 6, 7, 8 can be attached to the backing sheet 2 of the diaper 1, for example by ultrasonic welding, although other welding techniques or suitable adhesive may be used instead. Alternatively, the terminal portions may be formed continuously with the backing sheet 2 by cutting away a portion of the backing sheet 2 lying longitudinally between corresponding terminal portions of the front and back waist regions.

Before they are attached to the backing sheet 2, the terminal portions 5, 6, 7, 8 can be typically already folded.

A distal section 5a, 6a, 7a, 8a (that is a section furthest from the longitudinal centreline of the diaper 1) of each terminal portion 5, 6, 7, 8 is folded over a respective first fold line 9, 10, 11, 12 onto a middle section 5b, 6b, 7b, 8b of terminal portions 5, 6, 7, 8. Rows of ultrasonic spot welds 13 can then be formed to fasten the distal sections 5a, 6a, 7a, 8a to the middle sections 5b, 6b, 7b, 8b. These ultrasonic spot welds 13 are relatively weak so that the distal sections 5a, 6a, 7a, 8a may be pulled apart from the middle sections 5b, 6b, 7b, 8b without destroying the material of the terminal portions 5, 6, 7, 8.

The joined distal 5a, 6a, 7a, 8a and middle sections 5b, 6b, 7b, 8b are then folded over respective second fold lines 14, 15, 16, 17 onto proximal sections 5c, 6c, 7c, 8c of terminal portions 5, 6, 7, 8. Rows of ultrasonic spot welds 18 are then formed to fasten the distal sections 5a, 6a, 7a, 8a to the proximal sections 5c, 6c, 7c, 8c. These ultrasonic spot welds 18 also have the effect of fastening the distal sections 5a, 6a, 7a, 8a to the middle sections 5b, 6b, 7b, 8b as they are made through the thickness of the distal, middle and proximal sections. They are relatively weak so that the middle sections 5b, 6b, 7b, 8b may be pulled apart from the proximal sections 5c, 6c, 7c, 8c without destroying the material of the terminal portions 5, 6, 7, 8.

The distal sections 5a, 6a of the terminal portions 5, 6, of the back waist region have closure members 19a, 19b, 20a, 20b attached to them (for example, by ultrasonic welding). When the distal sections 5a, 6a of terminal portions 5, 6 are unfolded, the closure members 19a, 19b, 20a, 20b may be fastened to landing zones on terminal portions 7, 8 to hold the front and back waist regions together. Landing zones may also be provided on the central portion of the front waist region (i.e. the part lying between the terminal portions 7 and 8) where the closure members 19a, 19b, 20a, 20b may be fastened.

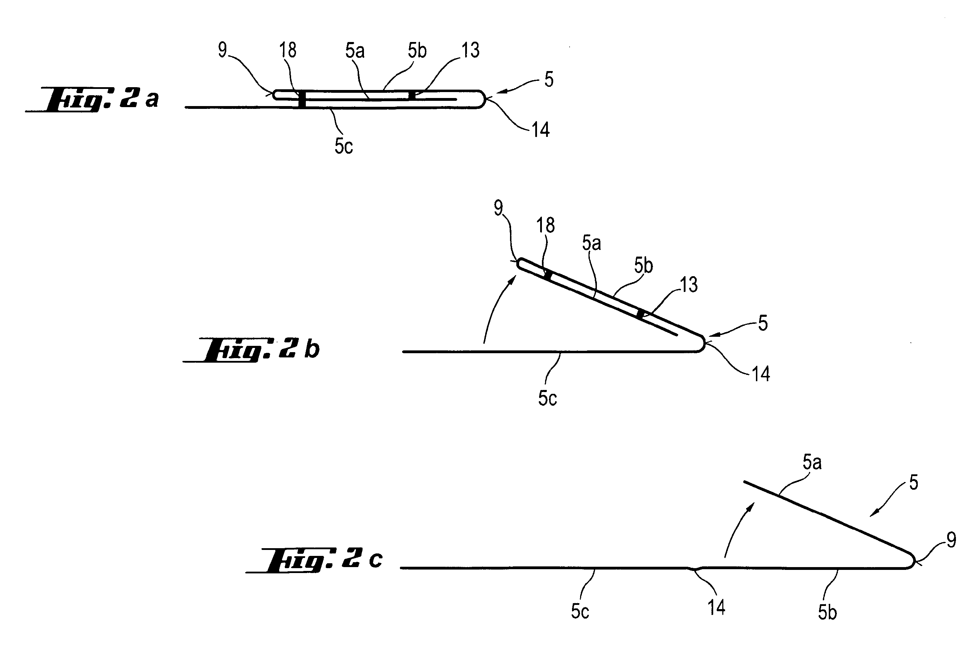

Figures 2a to 2c shows how one of the terminal portions 5 appears in cross-section in both its folded state, and as it is unfolded. In Figure 2a, the terminal portion 5 is folded, the distal section 5a lying between the middle section 5b and the proximal section 5c. The ultrasonic spot welds 13, 18 are visible. As mentioned, above spot welds 18 hold the distal section 5a to the proximal section 5c and to the middle section 5b.

By pulling the middle section 5b such that it unfolds around fold line 14, the spot welds 18 is broken between distal section 5a and proximal section 5c, which allows the joined middle 5b and distal sections 5a to be fully unfolded around fold line 14. This is shown in Figure 2b.

Then in Figure 2c, the distal section 5a is pulled such that it unfolds around fold line 9. This breaks the spot welds 13, thereby allowing the distal section 5a to be fully unfolded around fold line 9.

Thus, as can be seen from Figures 2a, 2b, and 2c, the length of terminal portion 5a (and of course the other terminal portions) can be left in the short configuration of Figure 2a, or by breaking spot welds 18 it can be lengthened to a medium configuration. Finally, by breaking spot welds 13 it can be lengthened again to a long configuration.

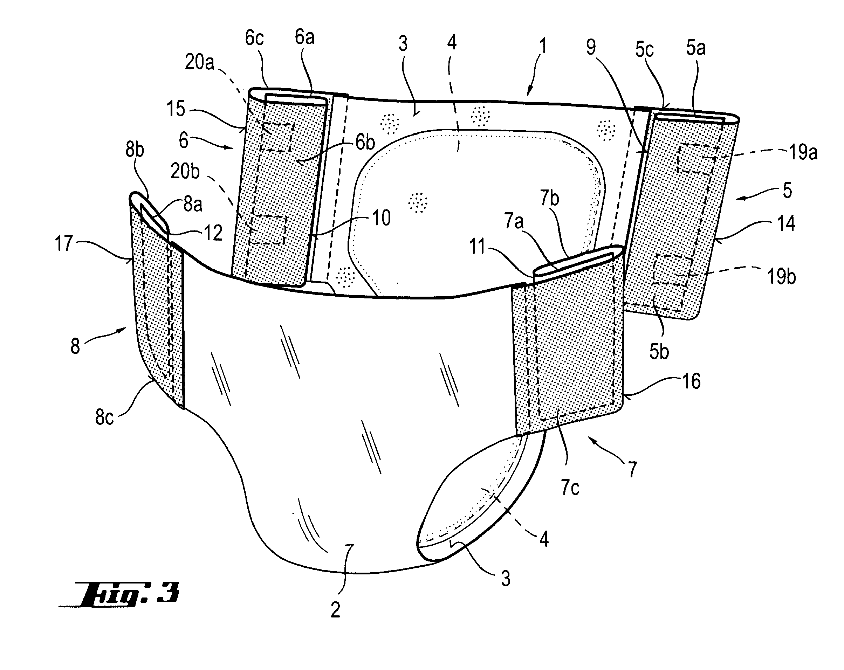

Figures 3 to 7 show the overall diaper 1 in various states as the terminal portions 5, 6, 7, 8 referred to above are unfolded. In Figure 3, all of the terminal portions 5, 6, 7, 8 are fully folded and the spot welds 13, 18 are intact. All that has occurred is that the terminal portions 5, 6, 7, 8, which are folded towards the longitudinal centreline of the diaper 1 during manufacture to lie over the portions of the front and back waist regions that do not extend transversely beyond the crotch region, have been unfolded.

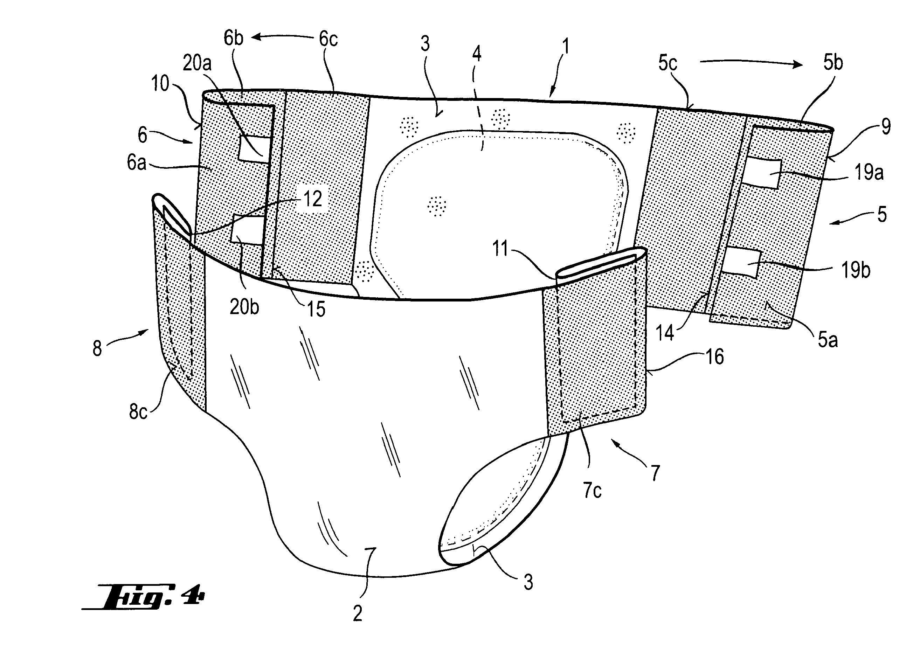

In Figure 4, the terminal portions 5, 6 of the back waist region are unfolded around fold line 14 and then fold line 9 by breaking spot welds 18 and 13 as explained above with reference to Figures 2a to 2c to open them out fully. This is necessary in this embodiment because the closure members 19a, 19b, 20a, 20b are attached to the distal sections 5a, 6a and are therefore only accessible when the terminal portions 5, 6 are fully unfolded.

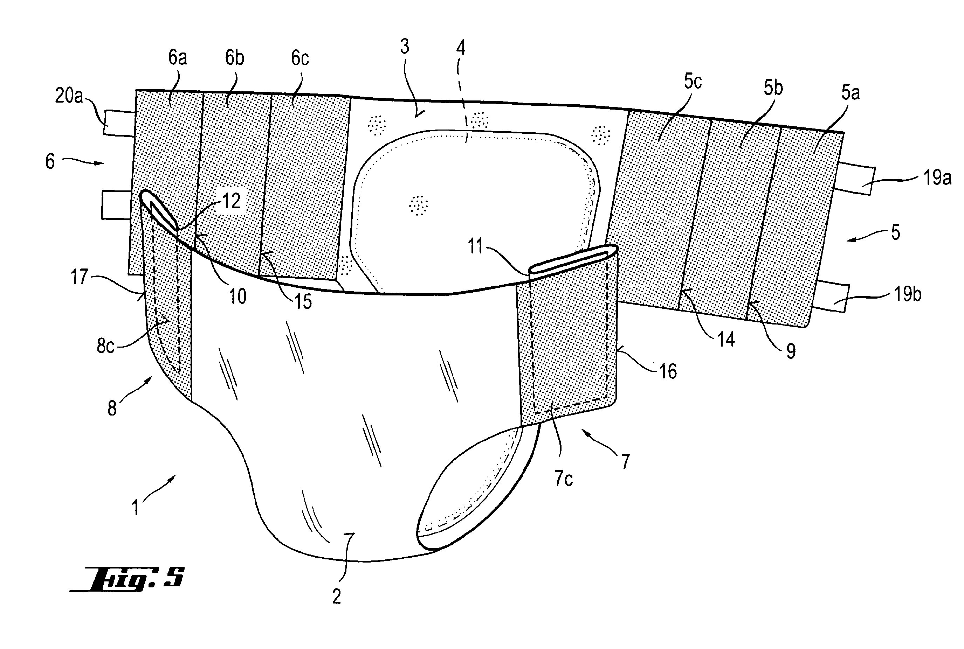

This configuration is shown in Figure 5. In Figure 5, the terminal portions 7, 8 of the front waist region have not been unfolded at all, and the spot welds 13, 18 of those terminal portions 7, 8 remain intact. In the configuration of Figure 5, the closure members 19a, 19b, 20a, 20b may be fastened to landing zones on proximal sections 7c, 8c of terminal portions 7, 8. The circumference around the front and back waist regions is relatively small, and would suit a wearer with a smaller waist.

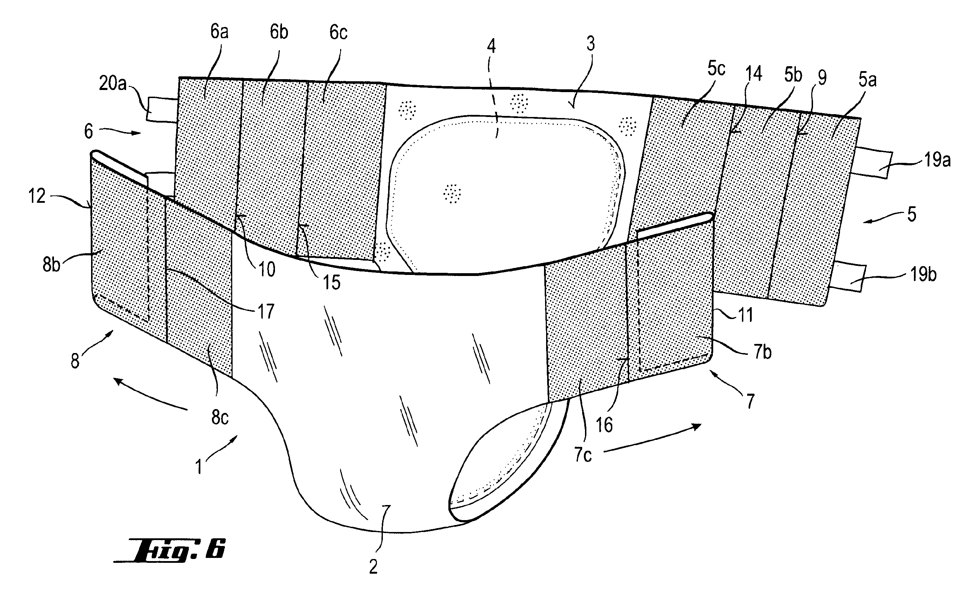

For a wearer with a larger waist size, the configuration of Figure 6 may be used. In this, the spot welds 18 between proximal 7c, 8c and middle sections 7b, 8b of terminal portions 7, 8 have been broken as shown in Figure 2b. The spot welds 13 and 18 between middle sections 7b, 8b and distal sections 7a, 8a of terminal portions 7, 8 remain intact, however. In this configuration, the closure members 19a, 19b, 20a, 20b may be fastened to landing zones on middle sections 7b, 8b of terminal portions 7, 8. This leads to a larger circumference around the front and back waist regions.

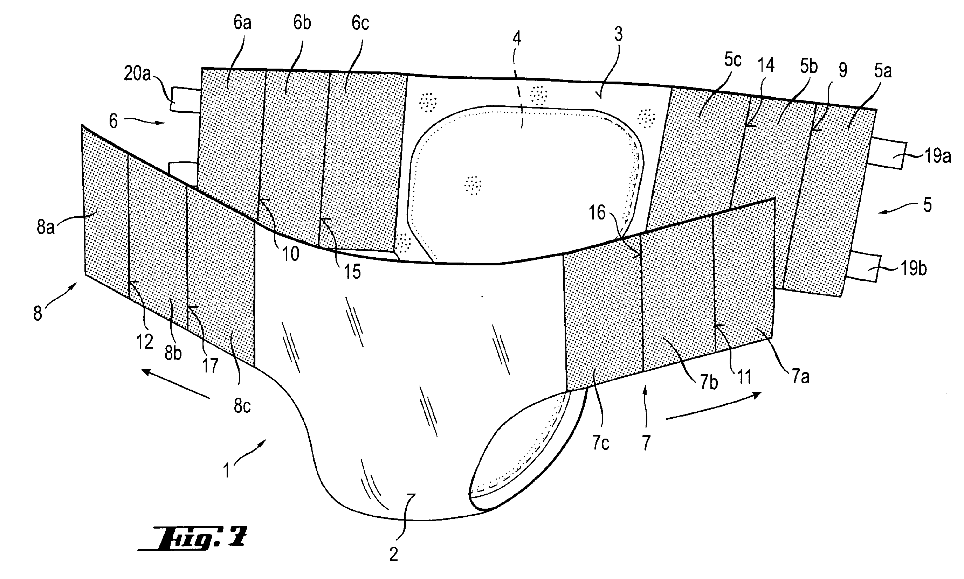

For wearers with even larger waist sizes, the configuration of Figure 7 may be used. In this, the spot welds 18 between proximal 7c, 8c and middle sections 7b, 8b of terminal portions 7, 8 have been broken as shown in Figure 2b. Furthermore, the spot welds 13 and 18 between middle sections 7b, 8b and distal sections 7a, 8a of terminal portions 7, 8 have also been broken as shown in Figure 2c. In this configuration, the closure members 19a, 19b, 20a, 20b may be fastened to landing zones on distal sections 7a, 8b of terminal portions 7, 8. This leads to the largest available circumference around the front and back waist regions.

Another embodiment is based on the embodiment described above with reference to Figures 1 to 7, but the terminal portions 7 and 8 are omitted. In this embodiment, the closure members 19a, 19b, 20a, 20b fasten directly to the central portion of the front waist region (i.e. the part lying between the terminal portions 7 and 8 in Figures 1 to 7). This embodiment of course provides the advantage of providing an additional degree of protection for the closure members mentioned above without providing the possibility of varying the degree of transverse extension of the terminal portions when the garment is put on a wearer.

Yet another embodiment is based on the embodiment described above with reference to Figures 1 to 7, but the terminal portions 5 and 6 are omitted and the closure members 19a, 19b, 20a, 20b are attached directly to the longitudinally-extending edges of the central portion of the back waist region (i.e. the part lying between the terminal portions 5 and 6 in Figures 1 to 7). This embodiment provides the advantage of varying the degree of transverse extension of the terminal portions when the garment is put on a wearer without providing the additional degree of protection for the closure members mentioned above.

The absorbent garments described above may be manufactured by modifying an existing manufacturing line for production of this type of article. The only additional step required is the folding of the terminal portions 5, 6, 7, 8. These may be folded prior to their attachment to the backing sheet 2. However, in an alternative construction, the terminal portions 5, 6, 7, 8 may be integral with the backing sheet 2, being formed by cutting the backing sheet 2 to a suitable shape.

## Claims

1. An absorbent garment comprising first and second waist regions and a crotch region lying longitudinally therebetween, the garment having a body-facing surface for facing a wearer and a garment-facing surface for facing away from the wearer, wherein the second waist region extends transversely beyond the crotch region to form terminal portions, each terminal portion of the second waist region being folded on at least two longitudinally-extending fold lines towards one of the body-facing and garment-facing surfaces of the garment, an outermost adjacent pair of fold lines dividing the terminal portions into proximal, middle and distal sections, the distal section lying between the proximal and middle sections, wherein at least one closure member for fastening to the first waist region is attached to the distal section of each terminal portion.

2. An absorbent garment according to claim 1, wherein the closure members are folded on the distal edge of the terminal portions of the second waist region towards the body-facing surface of the garment.

3. An absorbent garment according to claim 1 or claim 2, wherein the distal section of each terminal portion of the second waist region is releasably bonded to the middle and/or proximal sections.

4. An absorbent garment according to claim 3, wherein the distal section of each terminal portion of the second waist region is releasably bonded to the middle and/or proximal sections in two regions spaced apart in a longitudinal direction to leave an unbonded region therebetween.

5. An absorbent garment comprising first and second waist regions and a crotch region lying longitudinally therebetween, the garment having a body-facing surface for facing a wearer and a garment-facing surface for facing away from the wearer, wherein the first waist region extends transversely beyond the crotch region to form terminal portions and the second waist region has closure members for fastening to the terminal portions of the first waist region, each terminal portion of the first waist region being folded on at least two longitudinally-extending fold lines towards the body-facing surface of the garment and being divided into proximal, middle and distal sections by an outermost adjacent pair of the fold lines such that, when a terminal portion is unfolded on a first one of the pair of fold lines, a closure member can be fastened directly to the garment-facing surface on the middle section of the unfolded terminal portion, and when a terminal portion is unfolded on both of the pair of fold lines, a closure member can be fastened directly to the garment-facing surface on the distal section of the unfolded terminal portion.

6. An absorbent garment according to claim 5, the distal section of each terminal portion of the first waist region is releasably bonded to the middle and/or proximal sections.

7. An absorbent garment according to claim 6, wherein the distal section of each terminal portion of the first waist region is releasably bonded to the middle and/or proximal sections in two regions spaced apart in a longitudinal direction to leave an unbonded region therebetween.

8. An absorbent garment according to any of the preceding claims, wherein the first waist region is a front waist region and the second waist region is a back waist region.

9. An absorbent garment according to any of the preceding claims, wherein each of the terminal portions is provided as a discrete component that is attached to the first or second waist region.

10. An absorbent garment according to any of the preceding claims, wherein each of the terminal portions is folded along a respective longitudinally-extending line towards the body-facing surface to lie over a portion of the first or second waist region that does not extend transversely beyond the crotch region.

11. An absorbent garment according to any of the preceding claims, wherein the terminal portions of the first and/or second waist region are folded on only two longitudinally-extending fold lines.

12. An absorbent garment according to any of the preceding claims, wherein each terminal portion of the second waist region are folded on at least two longitudinally-extending fold lines towards the body-facing surface of the garment.

13. A method of using an absorbent garment according to any of claims 1 to 4 or any of claims 8 to 12 when dependent on any of claims 1 to 4, the method comprising placing the second waist region of the garment against a wearer's back, drawing the first waist region of the garment between the wearer's legs to leave the crotch region of the garment between the wearer's legs, placing the first waist region of the garment against a wearer's front, unfolding each terminal portion of the second waist region on each of the pair of adjacent longitudinally-extending fold lines, and fastening the closure members to the first waist region.

14. A method of using an absorbent garment according to any of claims 5 to 7 or any of claims 8 to 12 when dependent on any of claims 5 to 7, the method comprising placing the second waist region of the garment against a wearer's back, drawing the first waist region of the garment between the wearer's legs to leave the crotch region of the garment between the wearer's legs, placing the first waist region of the garment against a wearer's front, unfolding each terminal portion of the first waist region on a first one of the pair of adjacent longitudinally-extending fold lines, and fastening the closure members to the first waist region.

15. A method according to claim 14, further comprising unfolding each terminal portion of the first waist region on a second one of the pair of adjacent longitudinally-extending fold lines prior to fastening the closure members to the first waist region.
